# FASCICULE DE BREVET EUROPEEN

(11) **EP 0 420 706 B1**
(45) Date de publication et mention de la délivrance du brevet: **11.10.1995**
(21) Numéro de dépôt: 90401828.0
(22) Date de dépôt: 26.06.1990
(51) Int. Cl.: C07C 51/00, C07D 495/04

(54) **Procédé de préparation de dérivés phénylacétiques de thiénopyridines et des acides alpha-bromo phénylacétiques intermédiaires**
Verfahren zur Herstellung von Phenylessigsäurederivaten aus Thienopyridin und Zwischenprodukte von alpha-Bromphenylessigsäuren
Process for preparing phenylacetic derivatives of thienopyridines and intermediates alpha-bromo-phenylacetic acids

(30) Priorité: 29.09.1989 FR 8912787
(43) Date de publication de la demande: 03.04.1991
(73) Titulaire: SANOFI, 75008 Paris (FR)
(72) Inventeur: Bouisset, Michel, F-04200 Sisteron (FR); Radisson, Joel, F-31000 Toulouse (FR)
(74) Mandataire: Polus, Camille

(56) Documents cités:
- EP-A- 0 011 279
- EP-A- 0 099 802
- US-A- 2 778 851
- JOURNAL OF ORGANIC CHEMISTRY. WASHINGTON DC US pages 5214 - 5; W. REEVE ET AL.: 'Reactions of (Trihalomethyl)carbinols with Aquous Potassium Hydroxide'

## Description

La présente invention concerne un procédé de préparation de dérivés de thiéno[3,2-c] pyridine, dont le clopidogrel, et de leurs intermédiaires de synthèse, les acides α-bromo phénylacétiques de formule 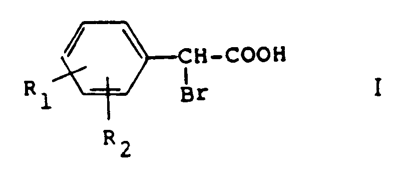
dans laquelle R₁ et R₂, identiques ou différents représentent l'atome d'hydrogène ou un atome d'halogène. Ces acides sont des produits connus, utilisés comme intermédiaires dans la synthèse de divers composés, notamment dans les industries pharmaceutiques et agrochimiques, éventuellement après estérification.

Ainsi l'acide α-chloro phénylacétique est mis en oeuvre, par exemple, dans la préparation des pénicillines semi-synthétiques décrites dans le brevet DE-A-2 624 064, des sels de thiazolium antiulcéreux décrits dans US-A-4 289 697, des sels de thiazolo[2,3-b]thiazolium, inhibiteurs de métastases, décrits dans US-A-4 327 221, ou encore de dérivés de thiéno[3,2-c]pyridine décrits dans EP-A-99802, de formule II 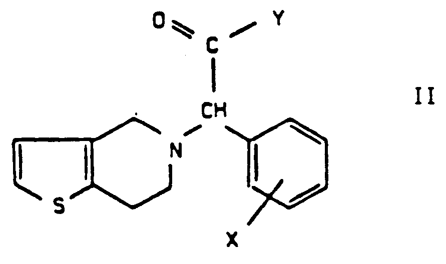
dans laquelle Y peut représenter l'hydroxyle OH ou le groupe OR dans lequel R est un radical alcoyle inférieur droit ou ramifié, ou bien Y représente un groupe NR₁R₂, dans lequel R₁ et R₂ sont chacun indépendamment l'un de l'autre l'hydrogène ou un groupe alcoyle inférieur droit ou ramifié, ou bien R₁ et R₂ forment ensemble et avec l'atome d'azote auquel ils sont rattachés un groupe pyrrolidino, morpholino, pipéridono ou benzyl-4 pipérazino, et X représente l'hydrogène, un halogène ou un radical alcoyle inférieur.

Le document J. Org. Chem. 45 (1980), pages 5214-5, décrit la préparation d'acide phénylacétique α-bromé à partir de dérivés (trihalométhyl)carbinol. Les rendements obtenus sont excessivement faibles et globalement inférieurs à 25%.

Le document EP-0 011 279 concerne la préparation d'acide arylacétique au départ d'un aldéhyde aromatique, d'un trihalométhane et d'un alcanethiol. Il résulte des exemples proposés dans ce document que la réaction est réalisée à une température au moins égale à la température ambiante.

Jusqu'à présent les composés de formule I ont été préparés, soit à partir des acides phénylacétiques correspondants par réaction avec la N-bromosuccinimide ou avec le brome, soit à partir des acides mandéliques par réaction avec une solution aqueuse concentrée d'acide bromhydrique. Les acides phénylacétiques sont eux-mêmes préparés, en plusieurs étapes, à partir du toluène, par l'intermédiaire du phénylacétonitrile, tandis que les acides mandéliques peuvent être préparés à partir des benzaldéhydes, sur lesquels on fait réagir soit un cyanure alcalin dans une réaction de Strecker pour obtenir le mandélonitrile qui est ensuite hydrolysé en milieu aqueux, soit le bromoforme en présence de potasse.

Ces procédés de préparation qui impliquent l'utilisation de réactifs coûteux ou dangereux ont des rendements faibles, et il était souhaitable de trouver un nouveau procédé pemettant de préparer dans des conditions économiques, notamment le composé antiagrégant plaquettaire de formule III 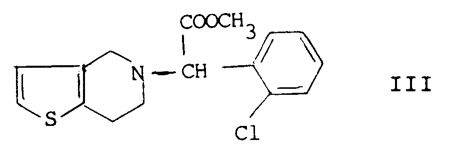
dont on connait la préparation par action de l'alphachloro (chloro-2) phénylacétate de méthyle sur la tétrahydrothiéno[3,2-c]pyridine avec des rendements moyens; il était en effet apparu, lors d'essais préléminaires, que le remplacement du dérivé alpha-chloré par un dérivé alpha-bromé pur permettait d'augmenter le rendement de la substitution de l'azote hétérocyclique conduisant au composé III.

L'invention permet de préparer en une seule étape, avec de bons rendements, les composés de formule I à partir des benzaldéhydes correspondants, en utilisant des réactifs commerciaux, qui peuvent être manipulés sans précautions spécifiques.

En effet, selon un premier aspect l'invention concerne un procédé de préparation d'acides alpha-bromophénylacétiques (I) qui consiste à faire réagir à une température comprise entre -5 et + 10°C un benzaldéhyde de formule 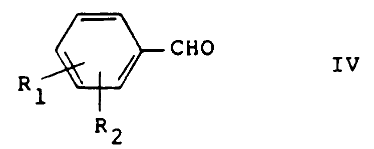
dans laquelle R₁ et R₂ ont la même signification que dans la formule I, avec du tribromométhane et l'hydroxyde de potassium dans l'eau, en présence d'un tiers solvant inerte.

Le solvant peut être un solvant miscible à l'eau, notamment un éther comme dioxanne, le diméthoxy-1,2 éthane, le diméthoxyméthane; le milieu réactionnel sera constitué d'un mélange à environ 50% en volume, de solvant et d'eau, mais il pourra être additionné d'eau en cours de réaction jusqu'à l'obtention d'un milieu contenant 90% d'eau.

Le solvant peut être aussi un solvant non miscible à l'eau comme un hydrocarbure aromatique, notamment le benzène, le toluène, ou un éther, comme l'éther isopropylique. Avec ces solvants, la réaction doit être effectuée en présence d'un catalyseur de transfert de phase, comme un sel d'ammonium quaternaire notamment un halogénure de tétrabutylammonium ou de benzyltriméthylammonium ou comme un sel de phosphonium.

On préfère faire réagir l'aldéhyde et le bromoforme en quantités sensiblement équivalentes, soit de 0,8 à 1,2 équivalent(s) de CHBr₃ et en présence de 3 à 4 équivalents de potasse. La concentration de KOH dans le milieu aqueux est généralement de 10 g à 50 g pour 100 ml.

Pour limiter notamment la formation d'acide, la réaction est effectuée à température assez basse, de préférence entre 0°C et 5°C. Le milieu réactionnel est maintenu sous agitation, selon la température, de plusieurs heures à quelques jours.

Lorsque la réaction est effectuée en milieu homogène, en fin de réaction, on lave la phase aqueuse par un solvant non miscible tel que l'éther isopropylique ou le dichlorométhane pour éliminer les impuretés, avant d'acidifier et d'extraire le produit final dans un solvant non miscible à l'eau, selon un procédé classique. L'acide mandélique, qui a pu se former, est séparé du dérivé alpha-bromé, par exemple en utilisant la différence entre leurs coefficients de partage entre le toluène et l'eau.

Le procédé convient tout particulièrement pour la préparation de composés de formule I dans laquelle R₁ = H et R₂ = Cl, Br ou F; les rendements en produits purs sont généralement supérieurs a 50% et fréquemment à 70%. On a, par contre, constaté qu'il ne donnait que des rendements très faibles lorsque le noyau aromatique était substitué par des groupes alkyle ou alkoxy. De même, il n'a pas été possible de remplacer le bromoforme par le chloroforme pour préparer dans les mêmes conditions les acides alphachlorés correspondants.

Un autre objet de l'invention est le procédé de préparation du composé de formule III et de ses sels pharmaceutiquement acceptables, qui consiste à faire réagir l'ester méthylique de l'acide alpha-bromo (chloro-2) phénylacétique avec la tétrahydro-4,5,6,7 thiéno[3,2-c]pyridine en présence d'au moins un équivalent d'une base dans un solvant polaire, usuel pour ce type de substitution, tel qu'un alcool, comme le méthanol ou l'éthanol, une cétone, comme l'acétone ou la méthyléthylcétone, un ester, comme l'acétate d'éthyle, un éther, comme le tétrahydrofuranne, ou l'éther isopropylique, l'acétonitrile, le diméthylformamide. Comme base, on met en oeuvre, de préférence, un carbonate alcalin tel que K₂CO₃, NaHCO₃ ou Na₂CO₃.

En fin de réaction, on filtre les solides et on évapore le solvant sous pression réduite. On peut alors préparer le chlorhydrate du produit final par action d'une solution aqueuse concentrée d'acide chlorhydrique sur l'amine en solution dans l'acétate d'éthyle.

On peut aussi faire réagir l'acide bromé sur l'hétérocyle azoté, avant d'estérifier le produit obtenu, mais on préfère préparer préalablement l'ester alpha-bromé, par exemple, par action de l'alcool sur l'acide bromé, en présence d'un acide fort.

Lorsque l'on veut préparer le clopidogrel (dénomination commune internationale), c'est-à-dire le stéréoisomère dextrogyre du composé de formule III, on effectue ensuite la recristallisation de sels de l'amine racémique avec un acide optiquement actif, tel que l'acide camphosulfonique, comme mentionné dans EP-A-0281459.

Dans ce qui suit, on décrit des exemples de mise en oeuvre de l'invention. Les produits obtenus sont analytiquement purs et leurs caractéristiques Physico-chimiques identiques à celles mentionnées dans la littérature.

### EXEMPLE 1 : acide α-bromo (chloro-2) phénylacétique.

On introduit sous forte agitation, à température voisine de 0°C, dans un mélange de 160 g de glace, 160 g d'hydroxyde de potassium et 100 ml de dioxanne, une solution de 100 g de chloro-2 benzaldéhyde et 198 g de tribromométhane dans 60 ml de dioxanne.

Au bout d'une heure, on introduit 1 litre d'eau et le milieu réactionnel est maintenu sous agitation durant 18 heures à une température comprise entre 0°C et 5°C. Il est ensuite lavé 3 fois avec 400 ml d'éther isopropylique froid.

La phase aqueuse est acidifiée par addition d'une solution aqueuse concentrée d'acide chlorhydrique, avant d'effectuer deux extractions par 400 ml de toluène. Le produit final est isolé de la phase toluénique après séchage et évaporation du solvant. Après recristallisation dans le toluène, on isole 111 g d'acide. Rendement 63% par rapport à l'aldéhyde.

### EXEMPLES 2 à 6

En appliquant le même mode opératoire, on a preparé les composés mentionnés dans le tableau I

**TABLEAU I**

| EXEMPLE | R₁ | R₂ | RENDEMENT |
|---|---|---|---|
| 2 | H | H | 51 % |
| 3 | H | Cl-4 | 74 % |
| 4 | H | Br-2 | 71 % |
| 5 | H | F-2 | 65 % |
| 6 | Cl-2 | Cl-4 | 68 % |

### EXEMPLE 7 : acide α-bromo (chloro-2) phénylacétique.

A température inférieure à 0°C, on ajoute sous forte agitation à une solution de 135 g de KOH et 12,5 g de chlorure de benzyltriméthylammonium dans 400 ml d'eau, une solution de 70,5 g de chloro-2 benzaldéhyde et 127 g de tribromométhane dans 150 ml d'éther isopropylique.

Le mélange est maintenu sous agitation, pendant 26 heures, à une température comprise entre - 5°C et 0°C. On ajoute alors 400 ml d'eau et 250 ml d'éther isopropylique et sépare la phase organique. Après lavage de la phase aqueuse avec 300 ml d'éther isopropylique, on l'acidifie par addition d'acide sulfurique concentré et sépare le produit final par deux extractions effectuées avec 400 ml d'éther isopropylique. Le solvant organique est alors évaporé et le résidu dissous dans 300 ml de toluène; la phase toluénique est lavée 2 fois avec 60 ml d'eau puis concentrée sous vide jusqu'à 60 ml. Le produit final précipite lentement dans ce milieu.

On isole ainsi 58,5 g de l'acide cherché - rendement 47%.

### EXEMPLE 8 : acide α-bromo (chloro-2) phénylacétique.

A une solution de 270 g de potasse et 34 g de bromure de tétrabutylphosphonium dans 800 ml d'eau, on ajoute une solution de 141 g de chloro-2 benzaldéhyde et 270 g de tribromométhane dans 500 ml d'éther éthylique. Le mélange est maintenu 24 heures, sous agitation, à 0°C.

Après traitement comme dans l'exemple 2, on obtient 119 g d'acide - rendement 48%-.

### EXEMPLE 9 : acide α-bromo (chloro-2) phénylacétique.

On introduit sous forte agitation à une température de -5°C environ dans un mélange de 131 ml d'eau, de 114 g de potasse (titre 86%), de 50 ml de diméthoxy-1,2 éthane, une solution de 70,3 g de chloro-2 benzaldéhyde et de 139 g de tribromométhane dilués dans 50 ml de diméthoxy-1,2 éthane. Après 3 heures à -5°C environ, on introduit dans le milieu réactionnel 500 ml d'eau à une température inférieure ou égale à 0°C, puis on élève la température jusqu'à + 10°C et la maintient durant 10 heures.

Le mélange réactionnel est ensuite extrait quatre fois par 95 ml de dichlorométhane. La phase aqueuse restante est alors acidifiée par addition d'acide chlorohydrique concentré puis extraite par deux fois 100 ml d'éther isopropylique. Après séchage et évaporation du solvant et recristallisation dans le toluène, on isole 84,6 g de l'acide attendu pur - rendement 67%.

On peut isoler un deuxième jet à partir de la solution toluénique, qui contient encore 14,5 g d'acide.

### EXEMPLE 10 : α-[tétrahydro-4,5,6,7 thiéno[3,2-c] pyridyl-5] (chloro-2)phénylacétate de méthyle.

a) 55 g d'acide α-bromo (chloro-2)phénylacétique obtenus à l'exemple 1 sont dissous dans 200 ml de méthanol; on ajoute alors 30 g d'acide sulfurique concentré et porte le milieu à sa température de reflux que l'on maintient 4 heures.
   On élimine alors le solvant sous pression réduite, et verse sur le résidu 100 ml d'éther isopropylique et 100 ml d'eau; après neutralisation, la phase éthérée est séchée, concentrée et l'ester méthylique distillé sous pression réduite pour donner 53,5 g de produit qui sera utilisé tel que dans l'étape suivante.
b) On introduit 7 g de tétrahydro-4,5,6,7 thiéno[3,2-c] pyridine pur, et 13,5 g d'α-bromo (chloro-2)phénylacétate de méthyle dans 80 ml de méthanol, avec 6 g de bicarbonate de sodium. Le milieu est maintenu 6 heures sous agitation à 80°C, puis les solides sont éliminés par filtration et le solvant évaporé sous pression réduite.
   On verse ensuite sur le résidu 120 ml d'acétate d'éthyle et 60 ml d'eau. La phase organique est décantée, lavée à l'eau puis refroidie vers -10°C. On y introduit alors un mélange de 20 g de glace et 10 ml d'acide chlorhydrique concentré. Le précipité qui se forme, est isolé par filtration et séché pour donner 15,8 g du chlorhydrate du produit cherché qui fond à 130°C.

## Revendications

1. Procédé de préparation de composés de formule I 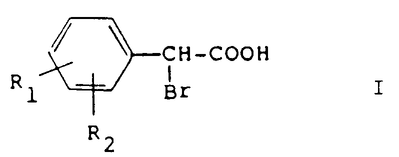 dans laquelle R₁ et R₂ identiques ou différents, représentent chacun l'hydrogène ou un halogène, caractérisé en ce que l'on fait réagir à une température comprise entre -5 et 10°C un aldéhyde de formule 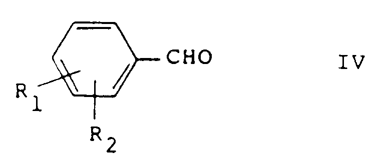 dans laquelle R₁ et R₂ ont la même signification que dans la formule I, avec CHBr₃ et KOH dans un mélange de solvant inerte et d'eau.

2. Procédé selon la revendication 1, caractérisé en ce que R₁ est H et R₂ est Cl-2.

3. Procédé selon l'une des revendications 1 à 2, caractérisé en ce que le solvant n'est pas miscible à l'eau et que la réaction est effectuée en présence d'un catalyseur de transfert de phase.

4. Procédé selon la revendication 3, caractérisé en ce que le solvant est choisi parmi le toluène et l'éther isopropylique.

5. Procédé selon l'une des revendications 1 ou 2, caractérisé en ce que le solvant est un éther miscible à l'eau.

6. Procédé de préparation d'un composé de formule III 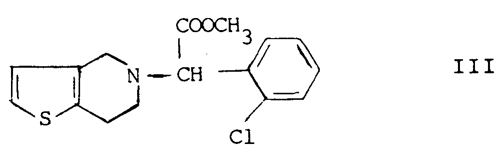 comprenant la réaction de l'ester méthylique de l'acide α-bromo(chloro-2)phénylacétique sur la tétrahydro-4,5,6,7 thiéno[3,2-c]pyridine en présence d'au moins un équivalent de base dans un solvant polaire, caractérisé en ce que l'acide α-bromo(chloro-2)phénylacétique est préparé selon le procédé de la revendication 1.

7. Procédé selon la revendication 6, caractérisé en ce que la base est choisie parmi K₂CO₃, NaHCO₃, Na₂CO₃.

## Claims

1. A process for the preparation of compounds of formula I 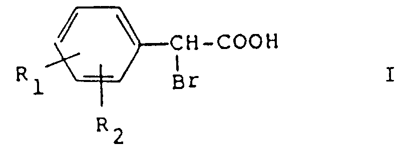 wherein R₁ and R₂, which may be identical or different, each represent hydrogen or a halogen, characterised in that an aldehyde of formula 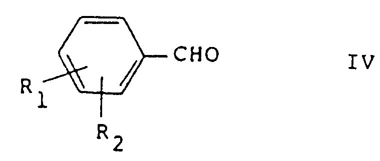 wherein R₁ and R₂ have the same meaning as in formula I above, is reacted with CHBr₃ and KOH at a temperature between -5 and 10°C in a mixture of inert solvent and water.

2. A process according to claim 1, characterised in that R₁ is H and R₂ is 2-chloro.

3. A process according to claim 1 or claim 2, characterised in that the solvent is not miscible with water, and in that the reaction is effected in the presence of a phase transfer catalyst.

4. A process according to claim 3, characterised in that the solvent is selected from toluene and isopropyl ether.

5. A process according to claim 1 or claim 2, characterised in that the solvent is an ether which is miscible with water.

6. A process for the preparation of a compound of formula III 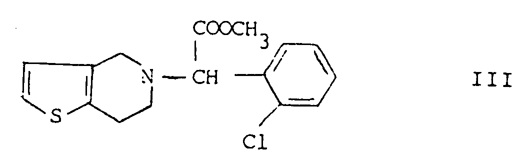 comprising reacting the methyl ester of α-bromo-(2-chloro)-phenylacetic acid with 4,5,6,7-tetrahydro-thieno[3,2-c]pyridine in the presence of at least one equivalent of base in a polar solvent, characterised in that the α-bromo-(2-chloro)-phenylacetic acid is prepared according to the process of claim 1.

7. A process according to claim 6, characterised in that the base is selected from K₂CO₃, NaHCO₃ and Na₂CO₃.

## Patentansprüche

1. Verfahren zur Herstellung der Verbindungen der Formel (I): 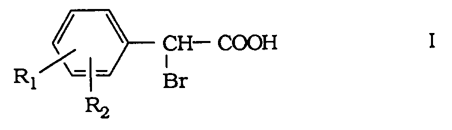 in der R₁ und R₂, die gleichartig oder verschieden sein können, jeweils Wasserstoff oder ein Halogen bedeuten, **dadurch gekennzeichnet**, daß man einen Aldehyd der Formel 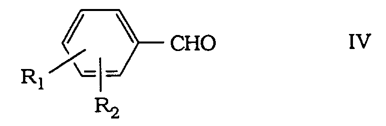 in der R₁ und R₂ die bezüglich der Formel I angegebenen Bedeutungen besitzen, bei einer Temperatur zwischen -5 und 10°C mit CHBr₃ und KOH in einer Mischung aus einem inerten Lösungsmittel und Wasser umsetzt.

2. Verfahren nach Anspruch 1, **dadurch gekennzeichnet**, daß R₁ H und R₂ 2-Cl bedeuten.

3. Verfahren nach einem der Ansprüche 1 bis 2, **dadurch gekennzeichnet**, daß das Lösungsmittel mit Wasser nicht mischbar ist und die Reaktion in Gegenwart eines Phasentransferkatalysators durchgeführt wird.

4. Verfahren nach Anspruch 3, **dadurch gekennzeichnet**, daß das Lösungsmittel aus Toluol und Isopropylether ausgewählt wird.

5. Verfahren nach einem der Ansprüche 1 oder 2, **dadurch gekennzeichnet**, daß das Lösungsmittel ein mit Wasser mischbarer Ether ist.

6. Verfahren zur Herstellung einer Verbindung der Formel III 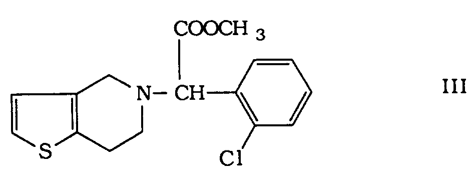 durch Umsetzen des Methylesters der α-Brom-(2-chlor)-phenylessigsäure mit 4,5,6,7-Tetrahydro-thieno[3,2-c]pyridin in Gegenwart mindestens eines Äquivalents einer Base in einem polaren Lösungsmittel, **dadurch gekennzeichnet**, daß die nach dem Verfahren gemäß Anspruch 1 hergestellte α-Brom-(2-chlor)-phenylessigsäure eingesetzt wird.

7. Verfahren nach Anspruch 6, **dadurch gekennzeichnet**, daß die Base aus K₂CO₃, NaHCO₃ und Na₂CO₃ ausgewählt wird.
